# EUROPEAN PATENT SPECIFICATION

(11) **EP 3 792 233 B1**
(45) Date of publication and mention of the grant of the patent: **11.05.2022**
(21) Application number: 20188602.5
(22) Date of filing: 30.07.2020
(51) Int. Cl.: C05F 17/60, C05F 17/929, C05F 17/979

(54) **FERMENTATION TREATMENT EQUIPMENT AND FERMENTATION TREATMENT METHOD FOR VARIOUS ANIMAL CARCASSES**
FERMENTATIONSBEHANDLUNGSANLAGE UND FERMENTATIONSBEHANDLUNGSVERFAHREN FÜR VERSCHIEDENE TIERKADAVER
ÉQUIPEMENT DE TRAITEMENT PAR FERMENTATION ET PROCÉDÉ DE TRAITEMENT PAR FERMENTATION POUR DIVERSES CARCASSES D'ANIMAUX

(30) Priority: 01.08.2019 CN 201910707777
(43) Date of publication of application: 17.03.2021
(73) Proprietor: Huang, Yung-Lin, 516100 Huizhou City Guangdong (CN)
(72) Inventor: Huang, Yung-Lin, 516100 Huizhou City Guangdong (CN)
(74) Representative: de Arpe Fernandez, Manuel

(56) References cited:
- CN-A- 109 848 178
- KR-B1- 102 036 091
- US-B1- 6 200 475

## Description

### TECHNICAL FIELD

The present disclosure relates to the fermentation field of various animal carcasses, in particular to a fermentation treatment equipment and a fermentation treatment method for various animal carcasses.

### BACKGROUND

The dead bodies of animals often carry a variety of pathogens. If not handled or handled improperly, the corpses will decompose quickly and reek, which will pollute the air, water and soil by pathogenic microorganisms, affect human living environment, and even cause the spread and propagation of diseases.

The most traditional methods of handling animal carcasses are direct landfill or cremation. There will be some invariable problems after the animal carcasses are buried, such as decomposition, putrefaction and reeking, which will lead to the pollution of air, water and soil. However, cremation directly results in the consumption of fuel and a lot of carbon emissions, which is not conducive to environmental protection.

Therefore, it has appeared in the market that high-temperature biodegradation method is used to treat animal carcasses, and finally to prepare organic fertilizer. However, this method is very energy-consuming, contrary to the concept of environmental protection, and can not achieve centralized control. Methods of converting organic waste by thermophilic fermentation are known from US6200475. Animal carcass microwave sterilization equipment and methods are also known from CN109848178.

### SUMMARY

In view of the above deficiencies, the present disclosure is to provide a fermentation treatment equipment and a fermentation treatment method for various animal carcasses, which integrates several fermentation tanks into one, and can simultaneously ferment multiple dead bodies separately to obtain separate organic fertilizer, which provides convenience for people to deal with animal carcasses, and reduces cost. Meanwhile, it is conducive to centralized control to the several fermentation tanks, so as to complete decomposition of animal carcasses with high efficiency, to achieve the object of energy conservation, carbon reduction, and achieve energy regeneration.

The technical solution adopted by the present disclosure to achieve the above object is as follows:
A fermentation treatment equipment for various animal carcasses, including a central controller, a centralized processing warehouse, a plurality of fermentation tanks set in the centralized processing warehouse, and at least one waste gas treatment device connected to the plurality of fermentation tanks, wherein the fermentation tank comprises a tank body base, a tank body arranged on the tank base, and a rotation driving mechanism which drives the tank body to rotate and is electrically connected to the central controller; an inner wall of the tank body is distributed with a plurality of heating components and a plurality of air supply components controlled by the central controller; a temperature sensor electrically connected to the central controller is provided in the tank body, and a rear end of the tank body is provided with an exhaust port connected to the waste gas treatment device.

As a further improvement of the present disclosure, the heating component includes a heating structure distributed on the inner wall of the tank body along a length direction of the tank body, and at least one heating element distributed in the heating structure along a length direction of the heating structure, wherein the heating element is connected to the the central controller via a power supply wire to realize power supply, or the heating element is powered by a microwave wireless power transmission device communicating with the central controller; the air supply component comprises an air pipe arranged on the inner wall of the tank body and distributed side by side with the heating structure, and a plurality of air holes are formed on side wall of the air pipe; a front end of the tank body is provided with a ventilation pipe connected between the air pipe and an external air supply device, and the ventilation pipe is provided with an electric control valve electrically connected to the central controller.

As a further improvement of the present disclosure, a plurality of strip grooves for embedding the heating component and the air supply component are formed on the inner wall of the tank body, and an insulating layer is provided on the inner wall of the tank body.

As a further improvement of the present disclosure, the heating component includes a printed copper foil attached to the inner wall of the tank body, the printed copper foil is connected to the central controller via a power supply wire to realize power supply, or the printed copper foil is powered by a microwave wireless power transmission device communicating with the central controller.

As a further improvement of the present disclosure, a plurality of conical breaking bumps are protruded arranged on the inner wall of the tank body.

As a further improvement of the present disclosure, the rotation driving mechanism includes a motor arranged on the tank body base and electrically connected to the central controller, a transmission shaft connected to an output shaft of the motor, and at least one transmission roller sleeved on a periphery of the transmission shaft and in contact with the outer wall of the tank body.

As a further improvement of the present disclosure, an exhaust structure is arranged at one end of the tank body near the exhaust port, the exhaust structure includes a baffle plate arranged in the tank body and a division plate arranged in the tank body between the baffle plate and the exhaust port, and an exhaust chamber is formed between the division plate and the exhaust port, an internal exhaust port is arranged on the baffle plate, an upper exhaust through hole and a lower exhaust through hole are formed between the division plate and the inner wall of the tank body; an upper exhaust channel and a lower exhaust channel are separately formed between the baffle plate and the division plate, wherein the upper exhaust channel is communicated between the internal exhaust port and the upper exhaust through hole, and the lower exhaust channel is communicated between the internal exhaust port and the lower exhaust through hole; and the internal exhaust port is respectively provided with a upper movable baffle swinging in the upper exhaust channel and a lower movable baffle swinging in the lower exhaust channel.

As a further improvement of the present disclosure, the tank body is mainly composed of a lower tank body and a upper cover covered on a lower tank body; a upper end of the lower tank body is provided with an open input port, and the upper cover is covered on the open input port; and the upper cover is provided with a plurality of hooks.

As a further improvement of the present disclosure, the waste gas treatment device includes an waste gas treatment tank, a plurality of waste gas ventilation pipes connected between a front end of the waste gas treatment tank and the plurality of fermentation tanks, and a negative pressure exhaust fan arranged at a rear end of the waste gas treatment tank, wherein an active bacteria bed is arranged at a front side of an interior of the waste gas treatment tank, a back side of the interior of the waste gas treatment tank is filled with activated carbon; an electric heating component is provided inside the waste gas ventilation pipe; the centralized treatment warehouse is provided with a solar photovoltaic panel which supplies power for the heating component, the electric heating component and the negative pressure exhaust fan, respectively.

A fermentation treatment method for animal carcasses according the above equipment, comprising:
(1) putting a single animal carcass into a numbered fermentation tank, adding the fermentation bacteria and filler, and covering up the fermentation tank;
(2) placing the fermentation tank on a shelf to a corresponding position of a centralized treatment warehouse;
(3) connecting a waste gas ventilation pipe of a waste gas treatment device to an exhaust port of the fermentation tank, and connecting an air pipe of the fermentation tank to an external air supply device;
(4) introducing air into into a tank body through the air pipe under a control of a central controller, and controlling internal heating components to heat a internal temperature of the tank body to 60°C - 70°C; wherein the central controller controls a rotation driving mechanism to drive the tank body to rotate at a slow speed for 10-30 days; during a rotation of the tank body, the fermentation bacteria in the tank body completely decompose the animal carcass to convert into biological energy, so as to form organic fertilizer and waste gas, and the waste gas is discharged from the exhaust port to the waste gas treatment device;
(5) purifying waste gas by the waste gas treatment device, and finally discharging the purified waste gas into an external environment.

As a further improvement of the present disclosure, the step (4) further includes the following steps: detecting, by a temperature sensor, the temperature inside the tank body in real time, and transmitting a detection result to the central controller in real time; disconnecting power supply for the heating component by the central controller when the temperature inside the tank body is detected to exceed 70 °C, and the heating component stops working; supplying power to the heating component by the central controller when the temperature inside the tank body is detected to lower than 60 °C, and the heating component start to work; repeating this step continuously to keep the temperature inside the tank body at 60 °C - 70 °C.

The advantageous effects of the present disclosure are as follows:
(1) Through the design of a centralized processing warehouse that can accommodate a plurality of fermentation tanks, the three-dimensional integration of the plurality of fermentation tanks can reduce the area occupied by fermentation tanks. At the same time, multiple animal carcasses can be fermented separately to obtain separate organic fertilizer, so that people can have equipment to deal with animal carcasses when people encounter animal carcasses, which provides great convenience for people to deal with all kinds of animal carcasses. Moreover, it is advantageous to centralized control and management of the plurality of fermentation tanks;
(2) The special structure design of a plurality of heating components and a plurality of air supply components in the fermentation tank can provide appropriate temperature and oxygen for the fermentation process in the tank, which is conducive to the decomposition and putrefaction of animal bodies inside the tank more completely and thoroughly, and finally to form organic fertilizer, which not only achieves the object of energy saving and carbon reduction, but also realizes energy regeneration;
(3) The waste gas produced by fermentation of the plurality of fermentation tanks is treated by the waste gas treatment device, and the toxic components and odors in the waste gas are absorbed by the active bacteria bed to complete once of the waste gas treatment process; then, the residual pollutants in the waste gas are absorbed by the activated carbon to separate the pollutants from the gas to obtain colorless, odorless and clean gas. And finally, the treated gas is discharged into the environment, and the waste gas treatment is complete and does no harm to the environment and human body;
(4) Through the real-time control of the temperature in the fermentation process, the internal temperature of the tank can be kept at 60 °C to 70 °C, which is conducive to the decomposition of animal carcasses by fermentation bacteria inside the tank;
(5) By controlling the rotation speed of the fermentation tank to rotate 2 to 3 turns a day, it not only consumes less electricity, but also saves energy and environmental protection. Due to the rotation of the fermentation tank, the animal carcass, fermentation bacteria and stuffing inside the fermentation tank can be constantly turned, so as to realize the internal oxygen supplement, which accelerates the whole aerobic fermentation process and improves the fermentation efficiency, thereby improving the turnover rate of each fermentation tank.
(6) The current energy consumption of the whole equipment is low, and solar power supply mode can be used to achieve the object of energy conservation and environmental protection.

The above is an overview of the technical solution of the present disclosure. The present disclosure is further described in combination with the attached drawings and specific embodiments.

### BRIEF DESCRIPTION OF THE DRAWINGS

Fig. 1 is a schematic diagram showing the overall structure of the present disclosure;
Fig. 2 is a schematic diagram showing the external structure of the fermentation tank in the present disclosure;
Fig. 3 is a schematic diagram showing the internal structure of the fermentation tank in the present disclosure;
Fig. 4 is a sectional view of a heating component and a gas supply component in the present disclosure;
Fig. 5 is a sectional view of the fermentation tank in the present disclosure;
Fig. 6 is an enlarged view of part A in Fig. 5;
Fig. 7 is the structural diagram of the conical breaking bumps in the tank body of the present disclosure;
Fig. 8 is a structural diagram of the exhaust structure in the present disclosure;
Fig. 9 is the structural diagram showing the upper cover of the tank body in the open state of the present disclosure.

### DETAILED DESCRIPTION OF THE EMBODIMENTS

In order to further illustrate the technical means and efficacy of the present disclosure to achieve the predetermined purpose, the specific implementation mode of the present disclosure is described in detail in combination with the attached drawings and preferred embodiments.

Referring to Fig.s 1 to 3, the present embodiment of the present disclosure provides a fermentation treatment device for various animal carcasses, including a central controller, a centralized processing warehouse 1, a plurality of fermentation tanks 2 arranged in the central processing warehouse 1, and at least one waste gas treatment device 3 connected to the plurality of fermentation tanks 2. Wherein, the fermentation tank 2 includes a tank base 21, a tank body 22 arranged on the tank base 21, and a rotation driving mechanism 23 which drives the tank body 22 to rotate and is electrically connected to the central controller. An inner wall of the tank body 22 is distributed with a plurality of heating components 24 and a plurality of air supply components controlled 25 by the central controller. The plurality of heating components 24 is used to heat the fermentation process in the tank body 22, and the plurality of air supply components controlled 25 provides oxygen for the fermentation process in the tank body 22, which is conducive to the decomposition of the animal carcasses inside the tank body more completely. At the same time, a temperature sensor electrically connected to the central controller is provided in the tank body 22, which is convenient for real-time detection to get the temperature in the tank body 22 and convenient for the control of the internal heating temperature. and a rear end of the tank body 22 is provided with an exhaust port 221 connected to the waste gas treatment device 3. After the fermentation is completed, the waste gas is discharged from the exhaust port 221, and finally the waste gas is carried out the purification treatment by the waste gas treatment device, and finally discharged to the external environment.

In the present embodiment, there are several positions for storing the fermentation tank 2 in the centralized processing warehouse 1. After the fermentation tank 2 is put into the position, the plurality of fermentation tanks 2 can be integrated into one, and multiple animal carcasses can be fermented separately at the same time to obtain separate organic fertilizer, so that people can have equipment to process animal carcasses when they encounter animal carcasses, providing a great convenience for people to handle animal carcasses, and reduces the cost. At the same time, the central controller can automatically and centrally control the work of the plurality of fermentation tanks 2, specifically to control the rotation speed and the internal heating temperature of the fermentation tank 2, and the air (oxygen) introduced into the tank body 22.

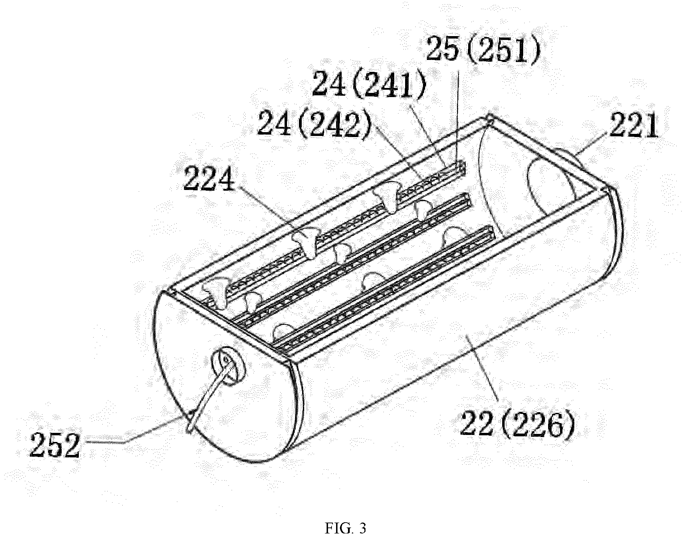

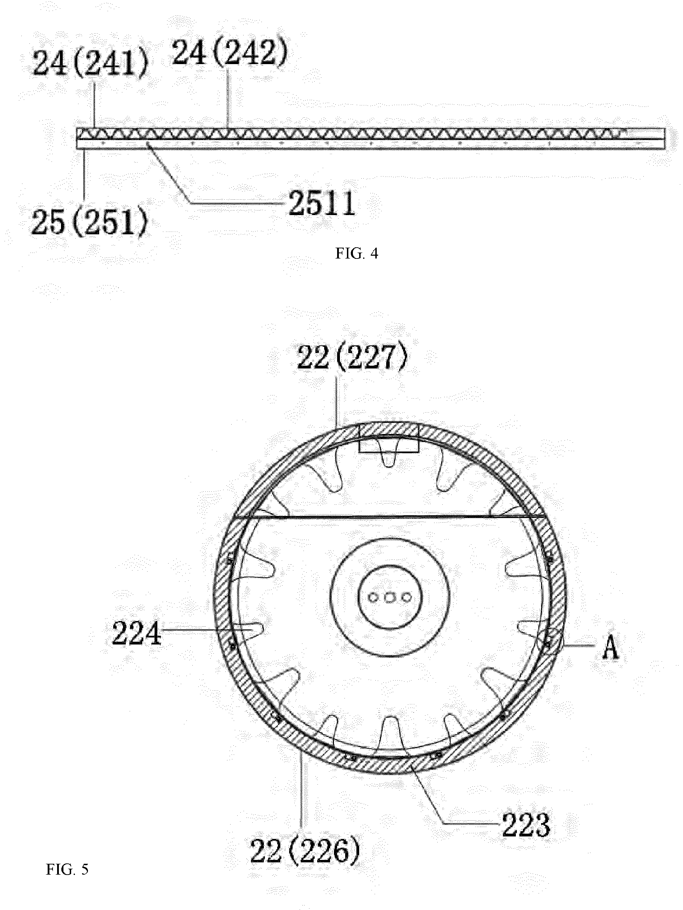

Specifically, as shown in Fig. 3 and Fig. 4, the heating component 24 includes a heating structure 241 distributed on the inner wall of the tank body 22 along the length direction of the tank body 22, and at least one heating element 242 distributed in the heating structure 241 along the length direction of the heating structure 241. Wherein, about the power supply of the heating element 242, it may be provided by wire power transmission mode or microwave wireless power transmission mode, no matter what power supply mode is selected, as long as the heating element 242 can be heated and the temperature in the tank body 22 can be increased. Specifically, the heating structure 241 can be a heating tube, and the heating element 242 can be a heating wire. Specifically, the wire power transmission mode is that the heating element 242 is connected to the the central controller via a power supply wire to realize power supply, while the microwave wireless power transmission mode is that the heating element 242 is powered by a microwave wireless power transmission device communicating with the central controller.

For the structure of the heating component 24, it further includes a printed copper foil attached to the inner wall of the tank body 22, the printed copper foil is connected to the central controller via a power supply wire to realize power supply, or the printed copper foil is powered by a microwave wireless power transmission device communicating with the central controller. The printed copper foil is electrified and heated to evenly heat the inside of the tank body 22, so as to increase the temperature in the tank body 22.

At the same time, the air supply component includes an air pipe 251 arranged on the inner wall of the tank body 22 and distributed side by side with the heating structure 241, and a plurality of air holes 2511 are formed on the side wall of the air pipe 251, as shown in Fig.4. At the front end of the tank body 22, there is a coupling device for the electrical structure and for oxygen to enter the tank body 22. The front end of the tank body 22 is provided with a ventilation pipe 252 connected between the air pipe 251 and an external air supply device, and the ventilation pipe 252 is provided with an electric control valve electrically connected to the central controller. The ventilation pipe 252 extends out through the coupling device. Air (oxygen) is introduced into the air pipe 251 by the external air supply device through the ventilation pipe 252, and the air is sprayed into the tank body 22 from the plurality of air holes 2511 on the side wall of the air pipe 251, so as to achieve preheating and accelerating the biodegradation reaction, which is conducive to the decomposition of animal carcasses by the fermentation bacteria inside the tank body.

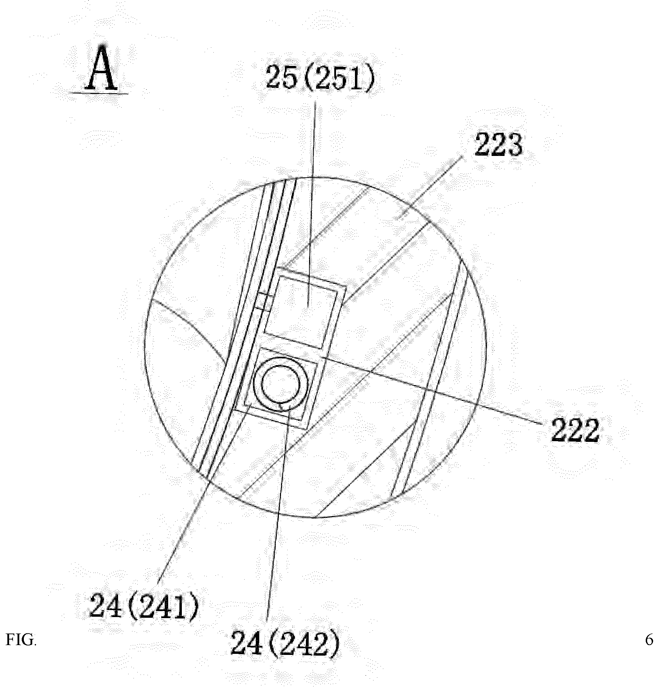

When the heating component 24 and the air supply assembly 25 are installed, a plurality of strip grooves 222 for embedding the heating component 24 and the air supply component 25 are formed on the inner wall of the tank body 22, as shown in Fig.5 and Fig.6. At the same time, an insulating layer 223 is provided on the inner wall of the tank body 22, which is beneficial to prevent the internal heat of the tank body 22 from being lost.

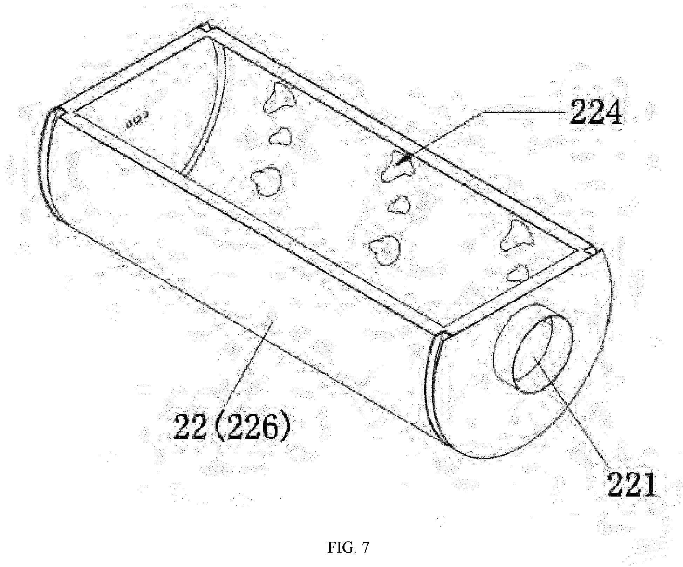

In order to accelerate the decomposition process of animal carcass, a plurality of conical breaking bumps 224 are protruded arranged on the inner wall of the tank body 22 in the present embodiment, as shown in Fig. 7. During the rotation of the tank body 22, the plurality of conical breaking bumps 224 collide and break the animal carcass and the filler, thus accelerating the decomposition process.

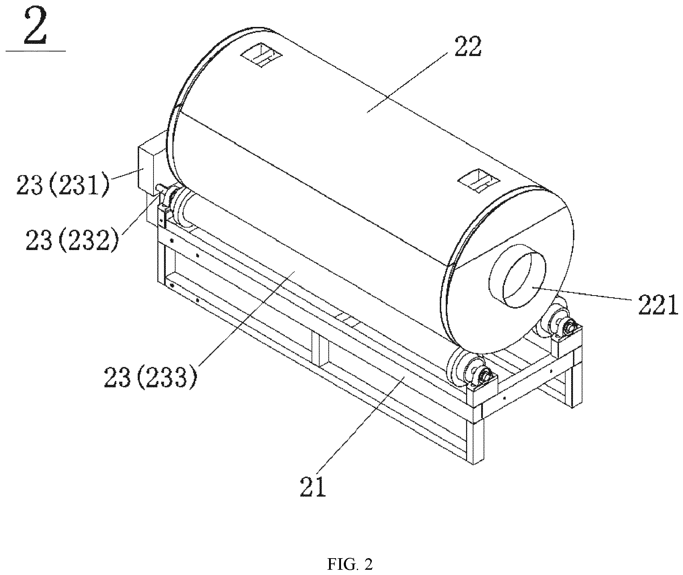

In the present embodiment, as shown in Fig. 2, the rotation driving mechanism includes a motor 231 arranged on the base of the tank body 22 and electrically connected to the central controller, a transmission shaft 232 connected to an output shaft of the motor 231, and at least one transmission roller 233 sleeved on the periphery of the transmission shaft 232 and in contact with the outer wall of the tank body 22. The start and stop of the motor 231 is controlled by the central controller. The motor 231 drives the transmission shaft 232 to rotate, the transmission roller 233 is driven to rotate under the rotation of the transmission shaft 232, and the rotation of the transmission roller 233 makes the tank body 22 rotate.

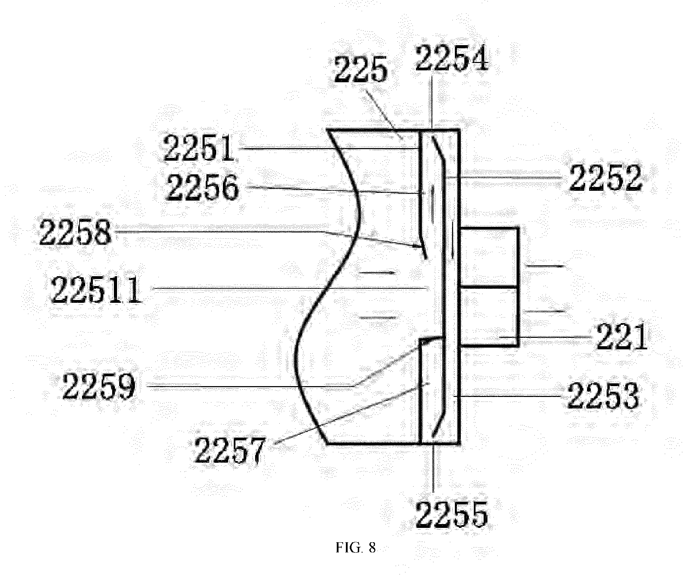

In the present embodiment, as shown in Fig.8, an exhaust structure 225 is arranged at one end of the tank body 22 near the exhaust port 221. Specifically, the exhaust structure 225 includes a baffle plate 2251 arranged in the tank body 22 and a division plate 2252 arranged in the tank body 22 between the baffle plate 2251 and the exhaust port 221, and an exhaust chamber 2253 is formed between the baffle plate 2251 and the exhaust port 221. An internal exhaust port 22511 is arranged on the baffle plate 2251, a upper exhaust through hole 2254 and a lower exhaust through hole 2255 are formed between the division plate 2252 and the inner wall of the tank body 22. An upper exhaust channel 2256 and a lower exhaust channel 2257 are separately formed between the baffle plate 2251 and the division plate 2252, wherein the upper exhaust channel 2256 is communicated between the internal exhaust port 22511 and the upper exhaust through hole 2254, and the lower exhaust channel 2257 is communicated between the internal exhaust port 22511 and the lower exhaust through hole 2255. At the same time, the internal exhaust port 22511 is respectively provided with a upper movable baffle 2258 swinging in the upper exhaust channel 2256 and a lower movable baffle 2259 swinging in the lower exhaust channel 2257.

In the present embodiment, the upper movable baffle 2258 and the lower movable baffle 2259 are movable plates which can swing freely, that is, when the tank body 22 rotates until the upper movable baffle 2258 is located above the lower movable baffle 2259, the downward swing of the upper movable baffle 2258 makes the internal exhaust port 22511 and the upper exhaust channel 2256 connect to each other, that is, the upper movable baffle 2258 is in the open state, and the waste gas can pass through. While the lower movable baffle 2259 is just against the division baffle 2252, in other words, the lower movable baffle 2259 is in a closed state, so that the internal exhaust port 22511 and the lower exhaust channel 2257 cannot be connected, and the waste gas cannot pass through. When the tank body 22 continues to rotate until the upper movable baffle 2258 is under the lower movable baffle 2259, similarly, the lower movable baffle 2259 swings down to connect the internal exhaust port 22511 and the lower exhaust channel 2257, that is, the lower movable baffle 2259 is in the open state, and the waste gas can pass through. While the upper movable baffle 2258 is just butted with the division plate 2252, that is, the upper movable baffle 2258 is in the closed state, so that the internal exhaust port 22511 is cannot be connected with the upper movable baffle 2258, so that the waste gas cannot pass through. Therefore, the waste gas is always discharged from the upper exhaust channel inside the tank body, while the lower exhaust channel is blocked to prevent the internal substances from entering or even blocking the lower exhaust channel.

After the waste gas produced during fermentation process enters the upper exhaust channel, it enters the exhaust chamber 2253 through the upper exhaust through hole, and is finally discharged from the exhaust port 221 to the waste gas treatment device 3.

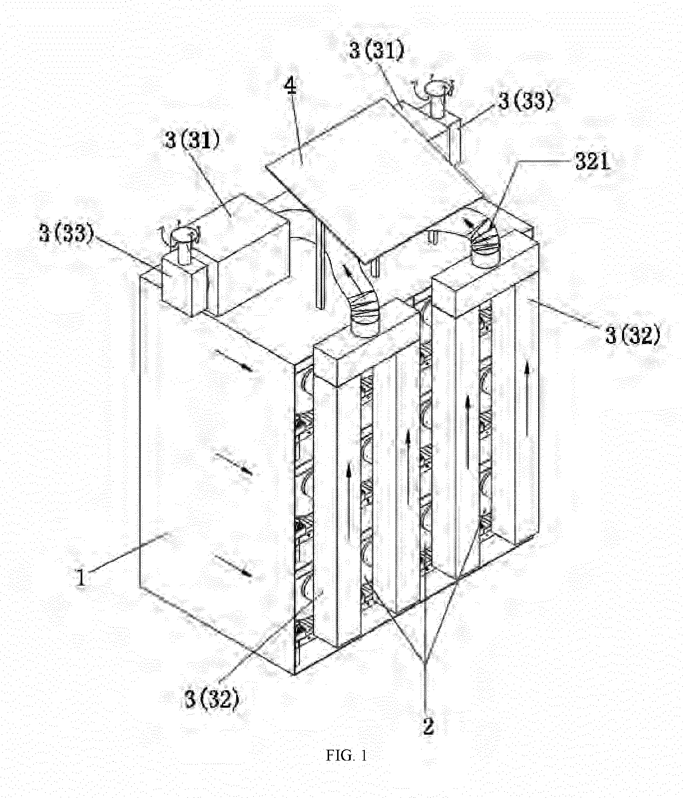

In the present embodiment, as shown in Fig.1, the waste gas treatment device 3 includes an waste gas treatment tank 31, a plurality of waste gas ventilation pipes 32 connected between the front end of the waste gas treatment tank 31 and the plurality of fermentation tanks 2, and a negative pressure exhaust fan 33 arranged at the rear end of the waste gas treatment tank 31. Wherein, an active bacteria bed is arranged at the front side of the interior of the waste gas treatment tank 31, the back side of the interior of the waste gas treatment tank 31 is filled with activated carbon. Specifically, the active bacteria bed is mainly composed of several microbial beds stacked up and down, and the biomembranes are arranged in the microbial beds. Specifically, the biomembranes include plastic carrier, sawdust and microbial active bacteria filled in the plastic carrier.

The waste gas entering the waste gas treatment device 3 enters the waste gas treatment tank 31 through the waste gas ventilation pipe 32, and then the biomembrane in the active bacteria bed in the waste gas treatment tank 31 adsorbs the toxic ingredients and odors in the waste gas to complete the primary treatment of the waste gas. Then, the waste gas after the first treatment passes through the activated carbon, and the pollutants in the primary treated waste gas are adsorbed on the surface of the activated carbon to separate the pollutants from the primary treated waste gas to obtain a colorless, odorless and clean gas. Finally, the treated waste gas is lead to the environment by a negative pressure exhaust fan 33, such that the waste gas treatment is complete and harmless to the environment and human body.

In order to prevent the steam condensation phenomenon in the waste gas ventilation pipe 32, an electric heating component 321 is arranged in the waste gas ventilation pipe 32 in this embodiment. Specifically, the electric heating component 321 can be an electric heating wire. The waste gas ventilation pipe 32 is electrified and heated by the electric heating component 321, so as to properly heat the waste gas ventilation pipe 32, improve the internal temperature, so that the occurrence of steam condensation can be effectively prevented.

In order to achieve the purpose of energy saving, this embodiment makes reasonable use of solar heat. The centralized treatment warehouse 1 is provided with a solar photovoltaic panel 4 which supplies power for the heating component 24, the electric heating component 321 and the negative pressure exhaust fan 33, respectively. The solar photovoltaic panel 4 absorbs sunlight heat, converts it into electrical energy, and supplies power for the entire equipment system.

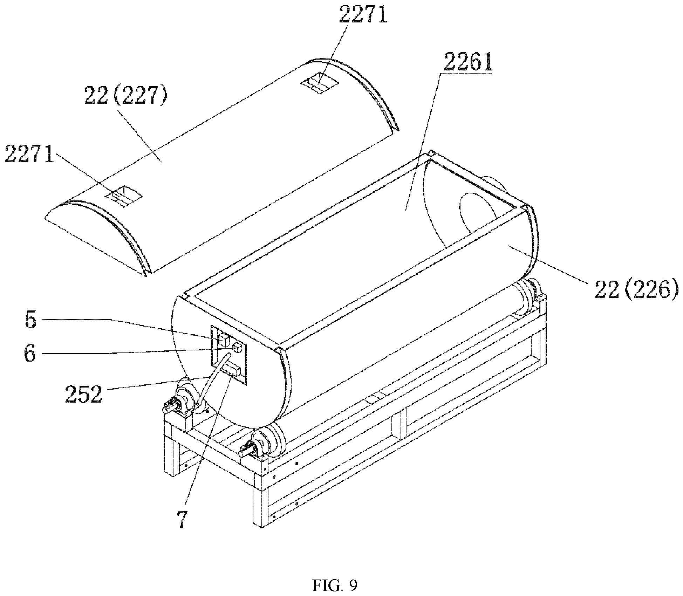

In order to facilitate to put the animal carcasses, filler and fermentation bacteria into the tank body 22, as shown in Fig.9, the tank body 22 is mainly composed of a lower tank body 226 and a upper cover covered 227 on a lower tank body 227 in the present embodiment. A upper end of the lower tank body is provided with an open input port 2261, and the upper cover 227 is covered on the open input port 2261. The animal carcasses, filler and fermentation bacteria are put into the tank body from the open input port 2261. Meanwhile, the upper cover 227 is provided with a plurality of hooks 2271, which is convenient for automatic lifting of the whole fermentation tank 2.

In order to ensure the tightness of the tank body 22 after the upper cover 227 is covered up the lower tank body 226, a sealing ring is arranged at the edge of the upper end surface of the lower tank body 226 in the present embodiment, so that the upper cover 227 is closely combined with the lower tank body 226, and the upper cover 227 is fixed to the lower tank body 226 by means of screws and other means.

The embodiment of the present disclosure further discloses a fermentation treatment method for various animal carcasses based on the above-mentioned equipment, including the following steps:
(1) Putting a single animal carcass into a numbered fermentation tank 2, adding the fermentation bacteria and filler, wherein the filler can be sawdust. And covering up the fermentation tank 2.
(2) Placing the fermentation tank 2 on a corresponding position of a centralized treatment warehouse 1. Automatic equipment can be used to lift the fermentation tank 2 to the position of centralized processing warehouse 1, and then the fermentation tank 2 can be automatic transferred into the corresponding position or pushed into the corresponding position by robot arm.
(3) Connecting a waste gas ventilation pipe of a waste gas treatment device to an exhaust port of the fermentation tank, and connecting an air pipe of the fermentation tank to an external air supply device.
(4) Introducing air into into a tank body through the air pipe under a control of a central controller, and controlling the heating components inside the tank body to heat the internal temperature of the tank body to 60°C - 70°C. At the same time, the central controller controls a rotation driving mechanism to drive the tank body to rotate at a slow speed for 10-30 days at a rotation rate of 0.083-0.125r/h. During the rotation of the tank body, the fermentation bacteria in the tank body completely decompose the animal carcass to convert into biological energy, so as to form organic fertilizer and waste gas, and the waste gas is discharged from the exhaust port to the waste gas treatment device.
(5) Purifying waste gas by the waste gas treatment device, and finally discharging the purified waste gas into an external environment.

The step (4) further includes the following steps: detecting, by a temperature sensor, the temperature inside the tank body in real time, and transmitting a detection result to the central controller in real time. When the temperature inside the tank body is detected to exceed 70 °C, the central controller disconnects the power supply for the heating component, and the heating component stops working. When the temperature inside the tank body is detected to lower than 60 °C, the central controller supplies power to the heating component, and the heating component start to work. Repeating the above step continuously to keep the temperature inside the tank body at 60 °C - 70 °C.

Of course, the embodiment can also set the corresponding thermometer 5, hygrometer 6 and time recorder 7 on the front end of the tank body 22 of the fermentation tank 2, and the thermometer 5 and the hygrometer 6 are inserted into the tank body 22, as shown in Fig. 9, so that the staff can watch the internal temperature, humidity and fermentation time of the tank body 22 in real time.

The fermentation treatment equipment for various animal carcasses provided by the embodiment can realize the automatic control and management of multiple fermentation tanks with numbers or labels in combination with the stereoscopic warehouse technology, so as to realize the following advantages:
(1) It is automatic operation, so as to improve efficiency and realize automatic storage and retrieval of fermentation tanks;
(2) It has accurate warehouse data management, such that the problem of inaccurate data can be solved, so as to reduce errors, providing more accurate warehouse management;
(3) It provides an accurate inquiry of the real time dynamic inventory information, data processing saves manpower;
(4) It can save storage space, so as to realize the rationalization of high-level warehouse, automatic storage and retrieval and easy operation.
(5) It can save energy and reduce carbon emissions.

The above is only a preferred embodiment of the present disclosure and does not limit the technical scope of the present disclosure. Therefore, other structures obtained by adopting the same or similar technical features as the above-mentioned embodiments of the present disclosure are within the scope of the present disclosure.

## Claims

1. A fermentation treatment equipment for various animal carcasses, comprising a central controller, a centralized processing warehouse, a plurality of fermentation tanks set in the centralized processing warehouse, and at least one waste gas treatment device connected to the plurality of fermentation tanks, wherein the fermentation tank comprises a tank body base, a tank body arranged on the tank base, and a rotation driving mechanism which drives the tank body to rotate and is electrically connected to the central controller; an inner wall of the tank body is distributed with a plurality of heating components and a plurality of air supply components controlled by the central controller; a temperature sensor electrically connected to the central controller is provided in the tank body, and a rear end of the tank body is provided with an exhaust port connected to the waste gas treatment device.

2. The fermentation treatment equipment for various animal carcasses according to claim 1, wherein the heating component comprises a heating structure distributed on the inner wall of the tank body along a length direction of the tank body, and at least one heating element distributed in the heating structure along a length direction of the heating structure, wherein the heating element is connected to the the central controller via a power supply wire to realize power supply, or the heating element is powered by a microwave wireless power transmission device communicating with the central controller; the air supply component comprises an air pipe arranged on the inner wall of the tank body and distributed side by side with the heating structure, and a plurality of air holes are formed on side wall of the air pipe; a front end of the tank body is provided with a ventilation pipe connected between the air pipe and an external air supply device, and the ventilation pipe is provided with an electric control valve electrically connected to the central controller.

3. The fermentation treatment equipment for various animal carcasses according to claim 2, wherein a plurality of strip grooves for embedding the heating component and the air supply component are formed on the inner wall of the tank body, and an insulating layer is provided on the inner wall of the tank body.

4. The fermentation treatment equipment for various animal carcasses according to claim 1, wherein the heating component comprises a printed copper foil attached to the inner wall of the tank body, the printed copper foil is connected to the central controller via a power supply wire to realize power supply, or the printed copper foil is powered by a microwave wireless power transmission device communicating with the central controller.

5. The fermentation treatment equipment for various animal carcasses according to claim 1, wherein a plurality of conical breaking bumps are protruded arranged on the inner wall of the tank body.

6. The fermentation treatment equipment for various animal carcasses according to claim 1, wherein the rotation driving mechanism comprises a motor arranged on the tank body base and electrically connected to the central controller, a transmission shaft connected to an output shaft of the motor, and at least one transmission roller sleeved on a periphery of the transmission shaft and in contact with the outer wall of the tank body.

7. The fermentation treatment equipment for various animal carcasses according to claim 1, wherein an exhaust structure is arranged at one end of the tank body near the exhaust port, the exhaust structure comprises a baffle plate arranged in the tank body and a division plate arranged in the tank body between the baffle plate and the exhaust port, and an exhaust chamber is formed between the division plate and the exhaust port, an internal exhaust port is arranged on the baffle plate, an upper exhaust through hole and a lower exhaust through hole are formed between the division plate and the inner wall of the tank body; an upper exhaust channel and a lower exhaust channel are separately formed between the baffle plate and the division plate, wherein the upper exhaust channel is communicated between the internal exhaust port and the upper exhaust through hole, and the lower exhaust channel is communicated between the internal exhaust port and the lower exhaust through hole; and the internal exhaust port is respectively provided with a upper movable baffle swinging in the upper exhaust channel and a lower movable baffle swinging in the lower exhaust channel.

8. The fermentation treatment equipment for various animal carcasses according to claim 1, wherein the tank body is mainly composed of a lower tank body and a upper cover covered on a lower tank body; a upper end of the lower tank body is provided with an open input port, and the upper cover is covered on the open input port; and the upper cover is provided with a plurality of hooks.

9. The fermentation treatment equipment for various animal carcasses according to claim 1, wherein the waste gas treatment device comprises an waste gas treatment tank, a plurality of waste gas ventilation pipes connected between a front end of the waste gas treatment tank and the plurality of fermentation tanks, and a negative pressure exhaust fan arranged at a rear end of the waste gas treatment tank, wherein an active bacteria bed is arranged at a front side of an interior of the waste gas treatment tank, a back side of the interior of the waste gas treatment tank is filled with activated carbon; an electric heating component is provided inside the waste gas ventilation pipe; the centralized treatment warehouse is provided with a solar photovoltaic panel which supplies power for the heating component, the electric heating component and the negative pressure exhaust fan, respectively.

10. A fermentation treatment method for animal carcasses according to any one of the equipment in claims 1 to 9, comprising:
(1) putting a single animal carcass into a numbered fermentation tank, adding the fermentation bacteria and filler, and covering up the fermentation tank;
(2) placing the fermentation tank on a shelf to a corresponding position of a centralized treatment warehouse;
(3) connecting a waste gas ventilation pipe of a waste gas treatment device to an exhaust port of the fermentation tank, and connecting an air pipe of the fermentation tank to an external air supply device;
(4) introducing air into into a tank body through the air pipe under a control of a central controller, and controlling internal heating components to heat a internal temperature of the tank body to 60°C - 70°C; wherein the central controller controls a rotation driving mechanism to drive the tank body to rotate at a slow speed for 10-30 days; during a rotation of the tank body, the fermentation bacteria in the tank body completely decompose and decay the animal carcass to convert into biological energy, so as to form organic fertilizer and waste gas, and the waste gas is discharged from the exhaust port to the waste gas treatment device;
(5) purifying waste gas by the waste gas treatment device, and finally discharging the purified waste gas into an external environment.

11. The fermentation treatment method for animal carcass according to claim 10, wherein the step (4) further comprises following steps: detecting, by a temperature sensor, the temperature inside the tank body in real time, and transmitting a detection result to the central controller in real time; disconnecting power supply for the heating component by the central controller when the temperature inside the tank body is detected to exceed 70 °C, and the heating component stops working; supplying power to the heating component by the central controller when the temperature inside the tank body is detected to lower than 60 °C, and the heating component start to work; repeating this step continuously to keep the temperature inside the tank body at 60 °C - 70 °C.

## Patentansprüche

1. Fermentationsbehandlungsanlage für verschiedene Tierkadaver, umfassend eine zentrale Steuerung, ein zentralisiertes Verarbeitungslager, eine Vielzahl von Fermentationsbehältern, die in dem zentralisierten Verarbeitungslager angeordnet sind, und mindestens eine Abgasbehandlungsvorrichtung, die mit der Vielzahl von Fermentationsbehältern verbunden ist, wobei der Fermentationsbehälter eine Behälterbasis, einen Behälterkörper, der auf der Behälterbasis angeordnet ist, und einen Drehantriebsmechanismus umfasst, der den Behälterkörper zum Drehen antreibt und elektrisch mit der zentralen Steuerung verbunden ist; Wobei auf einer Innenwand des Behälterkörpers eine Vielzahl von Heizkomponenten und eine Vielzahl von Luftzuführungskomponenten verteilt ist, die von der zentralen Steuerung gesteuert werden; wobei ein Temperatursensor, der elektrisch mit der zentralen Steuerung verbunden ist, in dem Behälterkörper vorgesehen ist, und wobei ein hinteres Ende des Behälterkörpers mit einer Abgasöffnung versehen ist, die mit der Abgasbehandlungsvorrichtung verbunden ist.

2. Fermentationsbehandlungsanlage für verschiedene Tierkadaver nach Anspruch 1, wobei die Heizkomponente eine Heizstruktur, die an der Innenwand des Behälterkörpers entlang einer Längsrichtung des Behälterkörpers verteilt ist, und mindestens ein Heizelement umfasst, das in der Heizstruktur entlang einer Längsrichtung der Heizstruktur verteilt ist, wobei das Heizelement mit der zentralen Steuerung über ein Stromversorgungskabel verbunden ist, um eine Stromversorgung zu realisieren, oder wobei das Heizelement durch eine drahtlose Mikrowellen-Stromübertragungsvorrichtung, die mit der zentralen Steuerung kommuniziert, mit Strom versorgt wird; wobei die Luftzuführungskomponente ein Luftrohr umfasst, das an der Innenwand des Behälterkörpers angeordnet und Seite an Seite mit der Heizstruktur verteilt ist, und wobei eine Vielzahl von Luftlöchern an einer Seitenwand des Luftrohrs ausgebildet ist; wobei ein vorderes Ende des Behälterkörpers mit einem Belüftungsrohr versehen ist, das zwischen dem Luftrohr und einer externen Luftzuführungsvorrichtung angeschlossen ist, und wobei das Belüftungsrohr mit einem elektrischen Steuerventil versehen ist, das elektrisch mit der zentralen Steuerung verbunden ist.

3. Fermentationsbehandlungsanlage für verschiedene Tierkadaver nach Anspruch 2, wobei an der Innenwand des Behälterkörpers eine Vielzahl von Streifennuten zur Einbettung der Heizkomponente und der Luftzuführungskomponente ausgebildet sind und eine Isolierschicht an der Innenwand des Behälterkörpers vorgesehen ist.

4. Fermentationsbehandlungsanlage für verschiedene Tierkadaver nach Anspruch 1, wobei die Heizkomponente eine gedruckte Kupferfolie umfasst, die an der Innenwand des Behälterkörpers angebracht ist, wobei die gedruckte Kupferfolie über ein Stromversorgungskabel mit der zentralen Steuerung verbunden ist, um eine Stromversorgung zu realisieren, oder wobei die gedruckte Kupferfolie durch eine drahtlose Mikrowellen-Energieübertragungsvorrichtung versorgt wird, die mit der zentralen Steuerung kommuniziert.

5. Fermentationsbehandlungsanlage für verschiedene Tierkadaver nach Anspruch 1, wobei eine Vielzahl von konischen Brechbeulen vorspringend an der Innenwand des Behälterkörpers angeordnet sind.

6. Fermentationsbehandlungsanlage für verschiedene Tierkadaver nach Anspruch 1, wobei der Drehantriebsmechanismus einen Motor, der an der Behälterbasis angeordnet und elektrisch mit der zentralen Steuerung verbunden ist, eine Übertragungswelle, die mit einer Ausgangswelle des Motors verbunden ist, und mindestens eine Übertragungsrolle umfasst, die auf einen Umfang der Übertragungswelle aufgesetzt ist und mit der Außenwand des Behälterkörpers in Kontakt steht.

7. Fermentationsbehandlungsanlage für verschiedene Tierkadaver nach Anspruch 1, wobei eine Abluftstruktur an einem Ende des Behälterkörpers in der Nähe der Abluftöffnung angeordnet ist, wobei die Abluftstruktur eine im Behälterkörper angeordnete Prallplatte und eine im Behälterkörper zwischen der Prallplatte und der Abluftöffnung angeordnete Teilungsplatte umfasst, wobei eine Abluftkammer zwischen der Teilungsplatte und der Abluftöffnung ausgebildet ist, wobei eine innere Abluftöffnung an der Prallplatte angeordnet ist, wobei ein oberes Abluftdurchgangsloch und ein unteres Abluftdurchgangsloch zwischen der Teilungsplatte und der Innenwand des Behälterkörpers ausgebildet sind; wobei ein oberer Abgaskanal und ein unterer Abgaskanal getrennt zwischen der Prallplatte und der Teilungsplatte ausgebildet sind, wobei der obere Abgaskanal zwischen der inneren Abgasöffnung und dem oberen Abgasdurchgangsloch und der untere Abgaskanal zwischen der inneren Abgasöffnung und dem unteren Abgasdurchgangsloch in Verbindung steht; und wobei die innere Abgasöffnung jeweils mit einer oberen beweglichen Prallplatte, die in dem oberen Abgaskanal schwingt, und einer unteren beweglichen Prallplatte, die in dem unteren Abgaskanal schwingt, versehen ist.

8. Fermentationsbehandlungsanlage für verschiedene Tierkadaver nach Anspruch 1, wobei der Behälterkörper hauptsächlich aus einem unteren Behälterkörper und einer oberen Abdeckung besteht, die auf dem unteren Behälterkörper abgedeckt ist; wobei ein oberes Ende des unteren Behälterkörpers mit einer offenen Eingangsöffnung versehen ist und die obere Abdeckung auf der offenen Eingangsöffnung abgedeckt ist; und wobei die obere Abdeckung mit einer Vielzahl von Haken versehen ist.

9. Fermentationsbehandlungsanlage für verschiedene Tierkadaver nach Anspruch 1, wobei die Abgasbehandlungsvorrichtung einen Abgasbehandlungsbehälter, eine Vielzahl von Abgasbelüftungsrohren, die zwischen einem vorderen Ende des Abgasbehandlungsbehälters und der Vielzahl von Fermentationsbehältern verbunden sind, und ein Unterdruckabzugsgebläse, das an einem hinteren Ende des Abgasbehandlungsbehälters angeordnet ist, umfasst, wobei ein aktives Bakterienbett an einer Vorderseite eines Innenraums des Abgasbehandlungsbehälters angeordnet ist, wobei eine Rückseite des Innenraums des Abgasbehandlungsbehälters mit Aktivkohle gefüllt ist; wobei eine elektrische Heizkomponente innerhalb des Abgasentlüftungsrohrs vorgesehen ist; wobei das zentrale Behandlungslager mit einem photovoltaischen Solarmodul versehen ist, das Strom für die Heizkomponente, die elektrische Heizkomponente bzw. den Unterdruckabzugsventilator liefert.

10. Verfahren zur Fermentationsbehandlung für verschiedene Tierkadaver nach einer der Anlage in den Ansprüchen 1 bis 9, umfassend:
(1) Einlegen eines einzelnen Tierkadavers in einen nummerierten Fermentationsbehälter, Hinzufügen der Fermentationsbakterien und des Füllstoffs und Abdecken des Fermentationsbehälters;
(2) Platzieren des Fermentationsbehälters auf einem Regal an einer entsprechenden Position eines zentralisierten Verarbeitungslager;
(3) Verbinden einer Abgasentlüftungsleitung einer Abgasbehandlungsvorrichtung mit einer Abluftöffnung des Fermentationsbehälters, und Verbinden einer Luftleitung des Fermentationsbehälters mit einer externen externen Luftzuführungsvorrichtung;
(4) Einleiten von Luft in einen Behälterkörpers durch das Luftrohr unter der Kontrolle einer zentralen Steuerung, und Steuern interner Heizkomponenten, um eine Innentemperatur des Behälterkörpers auf 60°C - 70°C zu erwärmen; wobei die zentrale Steuerung einen Rotationsantriebsmechanismus steuert, um den Behälterkörpers anzutreiben, sich mit einer langsamen Geschwindigkeit für 10-30 Tage zu drehen; während einer Rotation des Behälterkörpers die Fermentationsbakterien in dem Behälterkörpers den Tierkadaver vollständig zersetzen und verrotten lassen, um ihn in biologische Energie umzuwandeln, so dass organischer Dünger und Abgas gebildet werden, und das Abgas von der Abgasöffnung zu der Abgasbehandlungsvorrichtung abgeleitet wird;
(5) Reinigen des Abgases durch die Abgasbehandlungsvorrichtung und schließlich Ableiten des gereinigten Abgases in die Außenumgebung.

11. Verfahren zur Fermentationsbehandlung für verschiedene Tierkadaver nach Anspruch 10, wobei der Schritt (4) außerdem die folgenden Schritte umfasst: Erfassen der Temperatur im Inneren des Behälterkörpers durch einen Temperatursensor in Echtzeit und Übertragen eines Erfassungsergebnisses an die zentrale Steuerung in Echtzeit; Unterbrechen der Energieversorgung für die Heizkomponente durch die zentrale Steuerung und Aufhören der Heizkomponente, wenn festgestellt wird, dass die Temperatur im Inneren des Behälterkörpers 70 ° C überschreitet; Zuführen von Energie zu der Heizkomponente durch die zentrale Steuerung und Einschalten der Heizkomponente, wenn die Temperatur im Inneren des Behälterkörpers weniger als 60 °C ist; Wiederholen dieses Schrittes kontinuierlich, um die Temperatur im Inneren des Behälterkörpers auf 60 °C - 70 °C zu halten.

## Revendications

1. Équipement de traitement par fermentation pour des carcasses animaux, comprenant un contrôleur central, un entrepôt de traitement centralisé, une pluralité de cuves de fermentation installées dans l'entrepôt de traitement centralisé, et au moins un dispositif de traitement de gaz d'échappement connecté à la pluralité de cuves de fermentation, dans lequel la cuve de fermentation comprend une base de de cuve, un corps de cuve agencé sur la base de cuve et un mécanisme d'entraînement en rotation qui entraîne le corps de cuve en rotation et est électriquement connecté au contrôleur central ; une pluralité de composants de chauffage et une pluralité de composants d'alimentation en air sont réparties sur la paroi interne du corps de cuve et commandés par le contrôleur central ; un capteur de température est prévu dans le corps de cuve et électriquement connecté au contrôleur central, et une extrémité arrière du corps de cuve est pourvue d'un orifice d'échappement connecté au dispositif de traitement des gaz d'échappement.

2. Équipement de traitement par fermentation pour des carcasses animaux selon la revendication 1, dans lequel le composant de chauffage comprend une structure de chauffage disposée sur la paroi interne du corps de cuve le long d'une direction de longueur du corps de cuve, et au moins un élément de chauffage disposé dans la structure de chauffage le long d'une direction de longueur de la structure de chauffage, dans lequel l'élément de chauffage est connecté au contrôleur central par l'intermédiaire d'un fil électrique pour fournir une alimentation électrique, ou l'élément de chauffage est alimenté par un dispositif de transmission électrique sans fil à micro-ondes en communication avec le contrôleur central ; le composant d'alimentation en air comprend un tuyau d'air agencé sur la paroi interne du corps de cuve et disposé côte à côte avec la structure de chauffage, et une pluralité de trous d'air sont formés sur la paroi latérale du tuyau d'air ; le corps de cuve est pourvue sur une extrémité avant d'un tuyau de ventilation connecté entre le tuyau d'air et un dispositif d'alimentation en air externe, et le tuyau de ventilation est pourvue d'une valve de commande électrique qui est électriquement connectée au contrôleur central.

3. Équipement de traitement par fermentation pour des carcasses animaux selon la revendication 2, dans lequel une pluralité de rainures en bande dans lesquelles sont emboîtés le composant de chauffage et le composant d'alimentation en air sont formées sur la paroi interne du corps de cuve, et une couche isolante est prévue sur la paroi interne du corps de cuve.

4. Équipement de traitement par fermentation pour des carcasses animaux selon la revendication 1, dans lequel le composant de chauffage comprend une feuille de cuivre imprimée attachée à la paroi interne du corps de cuve, la feuille de cuivre imprimée étant connectée au contrôleur central par l'intermédiaire d'un fil électrique pour fournir une alimentation électrique, ou la feuille de cuivre imprimée étant alimentée par un dispositif de transmission électrique sans fil à micro-ondes en communication avec le contrôleur central.

5. Équipement de traitement par fermentation pour des carcasses animaux selon la revendication 1, dans lequel une pluralité de bosses de butée coniques sont disposées en saillie sur la paroi interne du corps de cuve.

6. Équipement de traitement par fermentation pour des carcasses animaux selon la revendication 1, dans lequel le mécanisme d'entraînement en rotation comprend un moteur agencé sur la base de corps de cuve et électriquement connecté au contrôleur central, un arbre de transmission connecté à un arbre de sortie du moteur, et au moins un galet de transmission manchonné sur une circonférence de l'arbre de transmission et en contact avec la paroi externe du corps de cuve.

7. Équipement de traitement par fermentation pour des carcasses animaux selon la revendication 1, dans lequel une structure d'échappement est agencée à une extrémité du corps de cuve près de l'orifice d'échappement, la structure d'échappement comprend une plaque de chicane agencée dans le corps de cuve et une plaque de division agencée dans le corps de cuve entre la plaque de chicane et l'orifice d'échappement, et une chambre d'échappement est formée entre la plaque de division et l'orifice d'échappement, un orifice d'échappement interne est agencé sur la plaque de chicane, un trou traversant d'échappement supérieur et un trou traversant d'échappement inférieur sont formés entre la plaque de division et la paroi interne du corps de cuve ; un canal d'échappement supérieur et un canal d'échappement inférieur sont séparément formés entre la plaque de chicane et la plaque de division, dans lequel le canal d'échappement supérieur est en communication entre l'orifice d'échappement interne et le trou traversant d'échappement supérieur, et le canal d'échappement inférieur est en communication entre l'orifice d'échappement interne et le trou traversant d'échappement inférieur ; et l'orifice d'échappement interne est pourvu respectivement d'un déflecteur mobile supérieur oscillant dans le canal d'échappement supérieur et d'un déflecteur mobile inférieur oscillant dans le canal d'échappement inférieur.

8. Équipement de traitement par fermentation pour des carcasses animaux selon la revendication 1, dans lequel le corps de cuve est essentiellement composé d'un corps de cuve inférieur et d'un couvercle supérieur recouvrant le corps de cuve inférieur ; une extrémité supérieure du corps de cuve inférieur est pourvue d'un orifice d'entrée ouvert, et le couvercle supérieur recouvre l'orifice d'entrée ouvert ; et le couvercle supérieur est pourvu d'une pluralité de crochets.

9. Équipement de traitement par fermentation pour des carcasses animaux selon la revendication 1, dans lequel le dispositif de traitement de gaz d'échappement comprend un cuve de traitement de gaz d'échappement, une pluralité de tuyaux de ventilation de gaz d'échappement connectés entre une extrémité avant de la cuve de traitement de gaz d'échappement et la pluralité de cuves de fermentation, et un ventilateur d'échappement à pression négative agencé à une extrémité arrière de la cuve de traitement de gaz d'échappement, dans lequel un côté avant d'un intérieur de la cuve de traitement de gaz d'échappement est pourvu d'un lit de bactéries actives, et un côté arrière de l'intérieur de la cuve de traitement de gaz d'échappement est rempli de charbon actif ; un composant de chauffage électrique est prévu à l'intérieur du tuyau de ventilation de gaz d'échappement ; l'entrepôt de traitement centralisé est pourvu d'un panneau solaire photovoltaïque qui alimente respectivement le composant de chauffage, le composant de chauffage électrique et le ventilateur d'échappement à pression négative.

10. Procédé de traitement par fermentation pour des carcasses animaux en utilisant l'équipement selon l'une quelconque des revendications 1 à 9, comprenant :
(1) placer une seule carcasse animale dans une cuve de fermentation numérotée, ajouter des bactéries de fermentation et une charge, et fermer la cuve de fermentation ;
(2) placer la cuve de fermentation sur une étagère à une position correspondante d'un entrepôt de traitement centralisé ;
(3) connecter un tuyau de ventilation de gaz d'échappement d'un dispositif de traitement de gaz d'échappement à un orifice d'échappement de la cuve de fermentation, et connecter un tuyau d'air de la cuve de fermentation à un dispositif d'alimentation en air externe ;
(4) introduire de l'air dans un corps de cuve par l'intermédiaire du tuyau d'air sous commande d'un contrôleur central, et commander des composants de chauffage internes pour chauffer le corps de cuve jusqu'à une température interne de 60°C - 70°C ; dans lequel le contrôleur central commande un mécanisme d'entraînement en rotation pour entraîner le corps de cuve en rotation à une basse vitesse pendant 10 à 30 jours ; pendant la rotation du corps de cuve, les bactéries de fermentation dans le corps de cuve décomposent et corrompent complètement la carcasse animale pour la convertir en énergie biologique, de manière à former des engrais organiques et de gaz d'échappement, et échapper le gaz d'échappement de l'orifice d'échappement vers le dispositif de traitement de gaz d'échappement ;
(5) purifier le gaz d'échappement par le dispositif de traitement de gaz d'échappement, et décharger enfin le gaz d'échappement purifié vers un environnement externe.

11. Procédé de traitement par fermentation pour des carcasses animaux selon la revendication 10, dans lequel l'étape (4) comprend en outre les étapes suivantes : détecter, par un capteur de température, une température à l'intérieur du corps de cuve en temps réel, et transmettre un résultat de détection au contrôleur central en temps réel ; mettre le composant de chauffage hors tension par le contrôleur central lorsque la température détectée à l'intérieur du corps de cuve dépasse 70 °C, le composant de chauffage cessant de fonctionner ; mettre le composant de chauffage sous tension par le contrôleur central lorsque la température détectée à l'intérieur du corps de cuve est inférieure à 60 °C, le composant de chauffage recommençant à fonctionner ; répéter cette étape en continu pour maintenir la température à l'intérieur du corps de cuve à 60 °C - 70 °C.
